# EUROPÄISCHE PATENTANMELDUNG

(11) **EP 3 336 117 A1**
(43) Veröffentlichungstag der Anmeldung: **20.06.2018**
(21) Anmeldenummer: 17192030.9
(22) Anmeldetag: 20.09.2017
(51) Int. Cl.: C08G 18/79, C09D 175/04, C08K 5/521, C08K 5/54, C07C 265/04, C07C 269/08, C08G 18/73, C08G 18/02

(54) **VERFAHREN ZUR HERSTELLUNG VON IN LÖSUNGSMITTELN FLOCKULATIONSSTABILEN POLYISOCYANATEN VON (CYCLO)ALIPHATISCHEN DIISOCYANATEN**

(71) Anmelder: BASF SE, 67056 Ludwigshafen am Rhein (DE)
(72) Erfinder: Schaefer, Harald, 67056 Ludwigshafen (DE); Genger, Thomas, 67056 Ludwigshafen (DE); Emmerling, Sebastian, 67056 Ludwigshafen (DE)
(74) Vertreter: BASF IP Association

(57) **Zusammenfassung**

Die vorliegende Erfindung betrifft ein neues Verfahren zur Herstellung von in Lösungsmitteln flockulationsstabilen, Isocyanuratgruppen aufweisenden Polyisocyanaten von (cyclo)aliphatischen Diisocyanaten.

## Beschreibung

Die vorliegende Erfindung betrifft ein neues Verfahren zur Herstellung von in Lösungsmitteln flockulationsstabilen Polyisocyanaten von (cyclo)aliphatischen Diisocyanaten.

Unter Flockulation im Sinne dieses Patents wird umfassend eine mit dem bloßen Auge erkennbare Feststoffbildung verstanden. Dies geht von einer leichten Trübung, feinen, erst durch rotierende Bewegung um die Längsachse des Lagergefäßes aufwirbelnden Niederschlägen, bis zur Bildung schwebender Flocken und schwerer Niederschläge.

Para-Toluolsulfonylisocyanat (H₃C(C₆H₄)SO₂-NCO) und Triethylorthoformat sind literaturbekannte Beispiele für Wasserfänger, die in Polyisocyanaten zur Stabilisierung eingesetzt werden. Diese Stabilisierung umfasst u.a. die Verhinderung der Flockulation aliphatischer Polyisocyanate in verdünnter Form in Lösungsmitteln wie sie für Lackanwendungen eingesetzt werden.
Wasser fangende reaktive Verbindungen reagieren statt der Isocyanat-Gruppen mit Wasser und verhindern, dass die Polyisocyanate zu Aminen hydrolysieren und diese mit weiterem Polyisocyanat zu hochfunktionellen (Poly)-Harnstoffpolyisocyanaten reagieren. Diese weisen eine schlechte Löslichkeit in Polyisocyanaten und deren Lösungen auf und können dabei Flocken und Niederschläge bilden.
Nachteilig an Wasserfängern ist, dass diese (mindestens) stöchiometrisch zur Menge des vorhandenen oder zu erwartenden Wassers zugegeben werden müssen. Bei para-Toluolsulfonylisocyanat entspricht dies der 12-fachen, bei Triethylorthoformat der 9-fachen Gewichtsmenge an Wasser. Herstellerseitig wird beim para-Toluolsulfonylisocyanat sogar die doppelte Äquivalentmenge gegenüber Wasser empfohlen, respektive Mengen an para-Toluolsulfonylisocyanat von 0,5-4,0 % und 1-3 % Triethylorthoformat auf das Gesamtgewicht der Formulierung. Werden solche Mengen eingesetzt, reduzieren sie allein durch den Verdünnungsfaktor entsprechend die NCO-Gruppen, was nachteilig für die Lackeigenschaften ist. Para-Toluolsulfonylisocyanat reagiert zu einem Sulfonamid und Kohlendioxid, gegebenenfalls unter Aufbau von Druck im Lagergefäß.

DE 124590 beschreibt die Anwendung von Sulfonylisocyanaten als wasserbindende Komponenten in Polyurethanpräpolymeren. EP 86871 beschreibt Nachteile dieser Verbindungen, da das durch Reaktion mit Wasser entstehende Tosylamid wegen seiner ausgeprägten Kristatallisationstendenz zur Stippenbildung im Lack führt. Zudem ist das Tosylisocyanat so hochreaktiv, dass es mit Wasser außerordentlich heftig reagiert.

JP4178370 B2 beschreibt eine Lösung bestehend aus einem NCO-terminierten Urethan-Präpolymeren, insbesondere auf Basis Toluoldiisocyanat, einem Phosphor- und/oder Phosphonsäuresilylester und Lösungsmittel, sowie einen Klebstoff oder eine Anstrichfarbe unter Verwendung der Lösung.
In den Beispielen werden konkret drei Toluoldiisocyanat-, ein Methylendiphenyl-, und ein Hexamethylen-diisocyanat-basiertes Präpolymer beschrieben.

US 6,291,577 offenbart eine Methode Feuchtigkeit in Polyisocyanatformulierungen einzufangen, indem man der Polyisocyanatformulierung Wasserfänger zumischt enthaltend a) Di-tert.-butylhydroxytoluol ausgewählt aus der Gruppe von 2,6-Ditert.-butyl-hydroxytoluol und 2-tert.-Butyl-hydroxytoluol und b) Alkylestern der Toluolsulfonsäure mit mindestens 90% Anteil an para-Alkylester, optional in wenigstens einem Lösungsmittel. Beispielsweise genannt sind ein Paket an Feuchtigkeitsfängern bestehend aus 0,19 % Bis-(tert.-butyl)-hydroxytoluol (BHT) und 1,0 % para-Toluolsulfonsäure-methylester, gegebenenfalls in Kombination mit anderen Feuchtigkeitsfängern, bezogen auf ein Isocyanuratgruppen aufweisendes Polyisocyanat auf Basis von Hexamethylendiisocyanat als 40 %-ige Lösung in n-Butylacetat. Entsprechende Mischungen sind 11 Wochen lagerstabil, ohne dass es zu Trübung oder Vergilbung kommt. Nachteilig an dem Verfahren ist, dass die Mengen an Feuchtigkeitsfänger sehr hoch sind, so dass der NCO-Wert des Polyisocyanats durch das Feuchtigkeitsfängerpaket durch Verdünnung um 1% reduziert wird. Die Löslichkeiten von para-Toluolsulfonsäuremethylester ist schlecht. Sie löst sich z.B. bei Raumtemperatur zwar 1 % in Butylacetat, aber nicht 10%-ig.

US 2008/0257214 beschreibt den Einsatz bestimmter Trimethylsilyl-Gruppen-haltiger Verbindungen wie Bistrimethylsilylacetamid oder Hexamethyldisilazan als Wasserfänger zum Verhindern von Trübungen und Kohlenstoffdioxidbildung von Polyisocyanaten in Lösungsmitteln. Wie bei den meisten Trockenmitteln müssen diese Verbindungen mindestens stöchiometrisch zugegeben werden. Die entstehenden Spaltprodukte verbrauchen NCO-Gruppen.

EP 203,874 offenbart Trialkyl-chloro-zinnverbindungen zur Stabilisierung von Polyisocyanaten in organischen Lösungsmitteln gegen Flockulat. Triorgano-Zinnverbindungen sind hochgradig toxisch.

Die WO 2013060809 beschreibt die Verwendung von Additiven ausgewählt aus der Gruppe bestehend aus
a1) organischen Säuren mit einem pKs-Wert von unter 4,2, ausgewählt aus der Gruppe bestehend aus a1a) aromatischen Sulfonsäuren und a1b) ein- oder zweifach mit Alkoxy-, Mercapto- oder Alkylmercapto-substituierte zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren
a2) Phosphiten der Formel 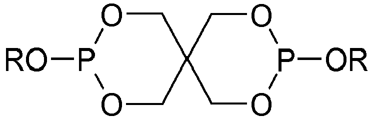 wobei R bevorzugt eine Aryl-Gruppe ist, die in 2, 4, und 6-Position wie folgt substituiert ist:
   Position 2: tert. Butyl, tert. Amyl
   Position 4: Wasserstoff, Alkyl, tert. Butyl oder tert. Amyl, und
   Position 6: Wasserstoff, Alkyl, tert. Butyl oder tert. Amyl,
   mit der Maßgabe, dass mindestens einer der Substituenten in Position 4 und 6 ungleich Wasserstoff ist,
a3) Phosphoniten der Formel (RO)₂P-X-P(RO)₂
   wobei R bevorzugt eine Aryl-Gruppe ist, die in 2, 4, und 6-Position wie folgt substituiert ist:
   Position 2: tert. Butyl, tert. Amyl
   Position 4: Wasserstoff, Alkyl, tert. Butyl oder tert. Amyl, und
   Position 6: Wasserstoff, Alkyl, tert. Butyl oder tert. Amyl,
   mit der Maßgabe, dass mindestens einer der Substituenten in Position 4 und 6 ungleich Wasserstoff ist, und X dabei eine Arylengruppe sei,
a4) sauren Phosphor-Derivaten, ausgewählt aus der Gruppe bestehend aus a4a) Mono- und Di-C₁- bis C₁₂-Alkylphosphaten, a4b) Mono- und Di-C₁- bis C₁₂-Alkylphosphonaten, a4c) Mono-C₁- bis C₁₂-Alkylphosphinaten und a4d) Alkylderivate von phosphorhaltigen Disäuren,
a5) blockierten aromatischen Sulfonsäuren
   zur Verringerung von Flockulation und/oder Niederschlagsbildung von Polyisocyanatgemischen, welche mindestens ein Lösungsmittel enthalten.
Nachteilig ist, dass die Flockulationsstabilität dieser Mischungen bei Lagerung noch nicht ausreichend ist, so dass weiterhin Bedarf an einer verbesserten Stabilisierung besteht.

Eine spezielle Problematik stellt sich beispielsweise für Refinish-Anwendungen (Automobil-Reparatur), bei denen von langen Lagerzeiten der Produkte in kleinen Gebinden auszugehen ist. Hersteller der Polyisocyanatkomponente in Lösungsmittel geben ihren Kunden Garantien des Beibehaltens der Produkteigenschaften von z.B. einem halben Jahr. Durch Lagerung beim Hersteller, Zwischenlagerung bei globalem Transport oder Überlagerung beim Kunden verlängern sich diese Fristen. Bedingt durch kleinvolumige Gebinde und/oder deren wiederholtes Öffnen kommt Umgebungsfeuchtigkeit an die Polyisocyanatkomponente. Ebenfalls enthalten die verwendeten Lösungsmittel Spuren an Feuchtigkeit. Lagereffekte werden insbesondere in südostasiatischen Ländern durch hohe Luftfeuchtigkeit und hohe Lagertemperaturen verschärft.

Generell ist bei allen Anwendungen bei denen längere Lagerzeiten auftreten, insbesondere in kleineren Gebinden, von der gleichen Problematik auszugehen, beispielsweise in manchen industriellen Anwendungen.

Aufgabe der vorliegenden Erfindung war es, ein Verfahren zur Verfügung zu stellen, mit dem Polyisocyanate während der Lagerung in Lösungsmitteln bei Exposition gegenüber (Luft-)Feuchtigkeit eine höhere Flockulationsstabilität bei Lagerung aufweisen, und die Additive in möglichst geringer Menge zugegeben werden müssen.

Die Aufgabe wurde gelöst durch die Verwendung von Additiven zur Verringerung von Flockulation und/oder Niederschlagsbildung von Polyisocyanatgemischen, welche mindestens ein Lösungsmittel (C) und mindestens ein Polyisocyanat (A) enthalten, wobei die Additive ausgewählt sind aus der Gruppe von Silylestern (B) bestehend aus Phosphorsäuresilylester und Phosphonsäuresilylester und in einer Menge von 1 bis 250 Gew.ppm bezogen auf das mindestens eine Polyisocyanat (A) eingesetzt werden.

Unter Polyisocyanaten (A) im Sinne der Erfindung werden Polyisocyanate wie sie bei Ihrer Synthese anfallen, respektive Gemische dieser Polyisocyanate verstanden. Diese umfassen die im Folgenden näher beschriebenen Verbindungen:
Die für die Herstellung der Polyisocyanate eingesetzten monomeren Isocyanate können aromatisch, aliphatisch oder cycloaliphatisch sein, bevorzugt aliphatisch oder cycloaliphatisch, was in dieser Schrift kurz als (cyclo)aliphatisch bezeichnet wird, besonders bevorzugt sind aliphatische Isocyanate.

Aromatische Isocyanate sind solche, die mindestens ein aromatisches Ringsystem enthalten, also sowohl rein aromatische wie auch araliphatische Verbindungen. Cycloaliphatische Isocyanate sind solche, die mindestens ein cycloaliphatisches Ringsystem enthalten.
Aliphatische Isocyanate sind solche, die ausschließlich gerade oder verzweigte Ketten enthalten, also acyclische Verbindungen.

Bei den monomeren Isocyanaten handelt es sich bevorzugt um Diisocyanate, die genau zwei Isocyanatgruppen tragen. Es kann sich aber prinzipiell auch um Monoisocyanate mit einer Isocyanatgruppe handeln.

Es kommen prinzipiell auch höhere Isocyanate mit im Mittel mehr als 2 Isocyanatgruppen in Betracht. Dafür eignen sich beispielsweise Triisocyanate wie Triisocyanatononan, 2'-Isocy-anatoethyl-(2,6-diisocyanatohexanoat), 2,4,6-Triisocyanatoluol, Triphenylmethantriisocyanat oder 2,4,4'-Trüsocyanatodiphenylether oder die Gemische aus Di-, Tri- und höheren Polyisocyanaten, die beispielsweise durch Phosgenierung von entsprechenden Anilin/Formaldehyd-Kondensaten erhalten werden und Methylenbrücken aufweisende Polyphenylpolyisocyanate darstellen, insbesondere Triisocyanatononan und 2'-Isocyanatoethyl-(2,6-diisocyanatohexa-noat.

Bei den monomeren Isocyanaten handelt es sich bevorzugt um Isocyanate mit 4 bis 20 C-Atomen. Beispiele für übliche Diisocyanate sind aliphatische Diisocyanate wie Tetramethylendiisocyanat, 1,5-Pentamethylendiisocyanat, Hexamethylendiisocyanat (1,6-Diisocyanatohexan), Octamethylendüsocyanat, Decamethylendiisocyanat, Dodecamethylendiisocyanat, Tetradecamethylendiisocyanat, Derivate des Lysindiisocyanats, (z.B. Methyl- oder Ethyl-2,6-diisocyanato-hexanoat), Trimethylhexandiisocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-me-thylcyclohexan sowie 3 (bzw. 4), 8 (bzw. 9)-Bis(isocyanatomethyl)-tricyclo[5.2.1.02.6]decan-Isomerengemische, sowie aromatische Diisocyanate wie 2,4- oder 2,6-Toluylendiisocyanat und deren Isomerengemische, m- oder p-Xylylendiisocyanat, 2,4'- oder 4,4'-Diisocyanatodiphenyl-methan und deren Isomerengemische, 1,3- oder 1,4-Phenylendiisocyanat, 1-Chlor-2,4-phenyl-endiisocyanat, 1,5-Naphthylendiisocyanat, Diphenylen-4,4'-diisocyanat, 4,4'-Diisocyanato-3,3'-dimethyldiphenyl, 3-Methyldi-phenylmethan-4,4'-diisocyanat, Tetramethylxylylendiisocyanat, 1,4-Diisocyanatobenzol oder Diphenylether-4,4'-diisocyanat.

Besonders bevorzugt sind 1,6-Hexamethylendiisocyanat, 1,5-Pentamethylendiisocyanat, 1,3-Bis(isocyanatomethyl)cyclo-hexan, Isophorondiisocyanat und 4,4'- oder 2,4'-Di(isocyanatocyclohexyl)methan, ganz besonders bevorzugt sind Isophorondiisocyanat und 1,6-Hexamethylendiisocyanat, insbesondere bevorzugt ist 1,6-Hexamethylendiisocyanat.

Es können auch Gemische der genannten Isocyanate vorliegen.

Isophorondiisocyanat liegt zumeist als ein Gemisch, und zwar der cis- und trans-Isomere vor, in der Regel im Verhältnis von ca. 60:40 bis 90:10 (w/w), bevorzugt von 70:30-90:10.
Dicyclohexylmethan-4,4'-diisocyanat kann ebenfalls als Gemisch der verschiedenen cis- und trans-Isomere vorliegen.

Für die vorliegende Erfindung können sowohl solche Diisocyanate eingesetzt werden, die durch Phosgenierung der korrespondierenden Amine erhalten werden, als auch solche, die ohne die Verwendung von Phosgen, d. h. nach phosgenfreien Verfahren, hergestellt werden. Nach Angaben der EP-A-0 126 299 (US 4 596 678), EP-A-126 300 (US 4 596 679) und EP-A-355 443 (US 5 087 739) beispielsweise können
(cyclo)aliphatische Diisocyanate, z.B. wie 1,6-Hexame-thylendiisocyanat (HDI), isomere aliphatische Diisocyanate mit 6 Kohlenstoffatomen im Alkylenrest, 4,4'- oder 2,4'-Di(isocyanatocyclohexyl)methan und 1-Isocyanato-3-isocyanato-methyl-3,5,5-trimethylcyclohexan (Isophorondiisocyanat bzw. IPDI) hergestellt werden durch Umsetzung der (cyclo)aliphatischen Diamine mit beispielsweise Harnstoff und Alkoholen zu (cyclo)-aliphatischen Biscarbaminsäureestern und deren thermische Spaltung in die entsprechenden Diisocyanate und Alkohole. Die Synthese erfolgt meist kontinuierlich in einem Kreislaufverfahren und optional in Gegenwart von N-unsubstituierten Carbaminsäureestern, Dialkylcarbonaten und anderen aus dem Reaktionsprozess zurückgeführten Nebenprodukten. So erhaltene Diisocyanate weisen in der Regel einen sehr geringen oder sogar nicht messbaren Anteil an chlorierten Verbindungen auf, was beispielsweise in Anwendungen in der Elektronikindustrie vorteilhaft ist.

In einer Ausführungsform der vorliegenden Erfindung weisen die eingesetzten Isocyanate an hydrolysierbarem Chlor weniger als 100 Gew.ppm auf, bevorzugt weniger als 50 Gew.ppm, insbesondere weniger als 30 ppm und speziell weniger als 20 Gew.ppm. Dies kann beispielsweise gemessen werden durch die ASTM-Vorschrift D4663-98. Die Gehalte an gesamtem Chlor liegen beispielsweise bei unter 1000 Gew.ppm, bevorzugt unter 800 Gew.ppm und besonders bevorzugt unter 500 Gew.ppm (ermittelt per argentometrischer Titration nach Hydrolyse).

Selbstverständlich können auch Gemische aus solchen monomeren Isocyanaten, die durch Umsetzung der (cyclo)aliphatischen Diamine mit beispielsweise Harnstoff und Alkoholen und Spaltung der erhaltenen (cyclo)aliphatischen Biscarbaminsäureester erhalten worden sind, mit solchen Diisocyanaten, die durch Phosgenierung der korrespondierenden Amine erhalten worden sind, eingesetzt werden.

Die Polyisocyanate (A), zu denen die monomeren Isocyanate oligomerisiert werden können, sind in der Regel wie folgt charakterisiert:
Die mittlere NCO Funktionalität solcher Verbindungen beträgt in der Regel mindestens 1,8 und kann bis zu 8 betragen, bevorzugt 2 bis 5 und besonders bevorzugt 2,4 bis 4.

Der Gehalt an Isocyanatgruppen nach der Oligomerisierung, berechnet als NCO = 42 g/mol, beträgt bevorzugt mindestens 15 Gew%, besonders bevorzugt mindestens 20 Gew%. Ganz besonders bevorzugt beträgt der Gehalt an Isocyanatgruppen nach der Oligomerisierung mindestens 20 Gew% und höchstens 30 Gew.%.

Bevorzugt handelt es sich bei den Polyisocyanaten (A) um folgende Verbindungen:
1) Isocyanuratgruppen aufweisende Polyisocyanate von aliphatischen und/oder cycloaliphatischen Diisocyanaten. Besonders bevorzugt sind hierbei Diisocyanate auf Basis von Hexamethylendiisocyanat und Isophorondiisocyanat. Bei den dabei vorliegenden Isocyanuraten handelt es sich insbesondere um Tris-isocyanatoalkyl- bzw. Tris-isocyanatocycloalkyl-Isocyanurate, welche cyclische Trimere der Diisocyanate darstellen, respektive um Gemische mit ihren höheren, mehr als einen Isocyanuratring aufweisenden Homologen. Die Isocyanato-Isocyanurate haben im Allgemeinen einen NCO-Gehalt von 15 bis 30 Gew.-%, insbesondere 15 bis 25 Gew.-% und eine mittlere NCO-Funktionalität von 2,6 bis 8. Die Isocyanuratgruppen aufweisenden Polyisocyanate können in geringerem Umfang auch Allophanat-und/oder Urethangruppen enthalten, bevorzugt mit einem Gehalt gebundenen Alkohols von kleiner 2 % bezogen auf das Polyisocyanat.
2) Uretdiongruppen aufweisende Polyisocyanate mit aliphatisch und/oder cycloaliphatisch gebundenen Isocyanatgruppen, insbesondere die von Hexamethylendiisocyanat oder Isophorondiisocyanat abgeleiteten. Bei Uretdiondiisocyanaten handelt es sich um cyclische Dimerisierungsprodukte von Diisocyanaten. Die Uretdiongruppen aufweisenden Polyisocyanate werden häufig im Gemisch mit anderen Polyisocyanaten, insbesondere den unter 1) genannten, erhalten. Uretdiongruppen aufweisende Polyisocyanate weisen üblicherweise Funktionalitäten von 2 bis 3 auf.
   Dies umfasst auch Uretdion-/Isocyanurat-Gemische beliebiger Zusammensetzung, insbesondere mit einem Gehalt an monomerem Uretdion (Dimer) von 1-40%, insbesondere 3-15, insbesondere 5-10 %.
   Dazu können die Diisocyanate unter Reaktionsbedingungen umgesetzt werden, unter denen sowohl Uretdiongruppen als auch die anderen Polyisocyanate gebildet werden, oder zunächst die Uretdiongruppen gebildet und diese anschließend zu den anderen Polyisocyanaten umgesetzt werden oder die Diisocyanate zunächst zu den anderen Polyisocyanaten und diese anschließend zu Uretdiongruppen-haltigen Produkten umgesetzt werden.
3) Biuretgruppen aufweisende Polyisocyanate mit cycloaliphatisch oder aliphatisch gebundenen Isocyanatgruppen, insbesondere Tris-(6-isocyanatohexyl)-biuret respektive dessen Gemische mit seinen höheren Homologen. Diese Biuretgruppen aufweisenden Polyisocyanate weisen im Allgemeinen einen NCO-Gehalt von 18 bis 24 Gew.-% und eine mittlere NCO-Funktionalität von 2,8 bis 6 auf.
4) Allophanat- und/oder Urethangruppen aufweisende Polyisocyanate mit aliphatisch oder cycloaliphatisch gebundenen Isocyanatgruppen, wie sie beispielsweise durch Umsetzung von überschüssigen Mengen an Diisocyanat, beispielsweise Hexamethylendiisocyanat, Pentamethylendiisocyanat oder Isophorondiisocyanat, mit ein- oder mehrwertigen Alkoholen entstehen. Diese Allophanat- und/oder Urethangruppen aufweisenden Polyisocyanate haben im Allgemeinen einen NCO-Gehalt von 15 bis 24 Gew.-% und eine mittlere NCO-Funktionalität von 2,0 bis 4,5. Solche Allophanat- und/oder Urethangruppen aufweisenden Polyisocyanate können unkatalysiert oder bevorzugt in Gegenwart von Katalysatoren, wie beispielsweise Ammoniumcarboxylaten oder -hydroxiden, oder Allophanatisierungskatalysatoren, z.B. Wismut-, Kobalt-, Cäsium-, Zn-(II)- oder Zr-(IV)-Verbindungen, jeweils in Anwesenheit von ein-, zwei- oder mehrwertigen, bevorzugt einwertigen Alkoholen, hergestellt werden.
   Diese Allophanat- und/oder Urethangruppen aufweisenden Polyisocyanate treten häufig in Mischformen mit den unter 1) genannten Polyisocyanaten auf.
5) Iminooxadiazindiongruppen enthaltende Polyisocyanate, vorzugsweise von Hexamethylendiisocyanat, Pentamethylendiisocyanat oder Isophorondiisocyanat abgeleitet. Solche Iminooxadiazindiongruppen enthaltenden Polyisocyanate sind aus Diisocyanaten mittels spezieller Katalysatoren, z.B. Phosphonium-hydrogendifluorid, herstellbar.
6) Hyperverzweigte Polyisocyanate, wie sie beispielsweise bekannt sind aus der DE-A1 10013186 oder DE-A1 10013187.
7) Die genannten Polyisocyanate können nach deren Herstellung in Biuretgruppen- oder Allophanat/Urethan-Gruppen aufweisende Polyisocyanate mit cycloaliphatisch oder aliphatisch gebundenen Isocyanatgruppen, überführt werden. Die Bildung von Biuretgruppen erfolgt beispielsweise durch Zugabe von Wasser oder Umsetzung mit Aminen. Die Bildung von Allophanat-/Urethangruppen erfolgt durch Umsetzung mit ein-, zwei- oder mehrwertigen, bevorzugt einwertigen Alkoholen, optional in Gegenwart von geeigneten Katalysatoren. Diese Biuret- oder Allophanat-/Urethangruppen aufweisenden Polyisocyanate weisen im Allgemeinen einen NCO-Gehalt von 15 bis 25 Gew.-% und eine mittlere NCO-Funktionalität von 3 bis 8 auf.
8) Modifizierte Polyisocyanate für Dual Cure Anwendungen, d.h. Polyisocyanate, die neben den unter 1) - 7) beschriebenen Gruppen solche enthalten, die formal durch Addition von Molekülen mit NCO-reaktiven Gruppen und durch UV- oder aktinische Strahlung vernetzbare Gruppen an die Isocyanatgruppen obiger Moleküle entstehen. Bei diesen Molekülen handelt es sich beispielsweise um Hydroxyalkyl(meth)acrylate und andere Hydroxy-Vinylverbindungen.

Die oben aufgeführten Diisocyanate oder Polyisocyanate können auch zumindest teilweise in blockierter Form vorliegen.

Zur Blockierung eingesetzte Verbindungsklassen sind beschrieben in D. A. Wicks, Z. W. Wicks, Progress in Organic Coatings, 36, 148-172 (1999), 41, 1-83 (2001) sowie 43, 131-140 (2001).

Beispiele für zur Blockierung eingesetzte Verbindungsklassen sind Phenole, Imidazole, Triazole, Pyrazole, Oxime, N-Hydroxyimide, Hydroxybenzoesäureester, sekundäre Amine, Lactame, CH-acide cyclische Ketone, Malonsäureester oder Alkylacetoacetate.

In einer bevorzugten Ausführungsform der vorliegenden Erfindung ist das Polyisocyanat (A) ausgewählt aus der Gruppe bestehend aus Isocyanuraten, Iminooxadiazindionen, Biureten, Urethanen und Allophanaten, bevorzugt aus der Gruppe bestehend aus Isocyanuraten, Urethanen und Allophanaten, besonders bevorzugt handelt es sich um ein isocyanuratgruppenhaltiges Polyisocyanat.

In einer besonders bevorzugten Ausführungsform handelt es sich bei dem Polyisocyanat (A) um Isocyanuratgruppen enthaltende Polyisocyanate von 1,6-Hexamethylendiisocyanat.

In einer weiteren bevorzugten Ausführungsform handelt es sich bei dem Polyisocyanat (A) um ein Gemisch von Isocyanuratgruppen enthaltenden Polyisocyanaten, ganz besonders bevorzugt von 1,6-Hexamethylendiisocyanat und von Isophorondiisocyanat.

In einer besonders bevorzugten Ausführungsform handelt es sich bei dem Polyisocyanat (A) um ein vornehmlich Isocyanuratgruppen enthaltendes Polyisocyanat, mit einer Viskosität von 500-4000 mPa*s, und/oder um ein niederviskoses Allophanat gegebenenfalls enthaltend Isocyanurat und/oder Urethan, mit einer Viskosität von 150-1600 mPa*s.

In dieser Schrift wird die Viskosität bei 23 °C gemäß DIN EN ISO 3219/A.3 in einem Kegel-Platte-System mit einem Geschwindigkeitsgefälle von 1000 s⁻¹ angegeben, falls nicht anders vermerkt.

Das Verfahren zur Herstellung der Polyisocyanate (A) kann erfolgen, wie in WO 2008 / 68198 beschrieben, dort besonders von Seite 20, Zeile 21 bis Seite 27, Zeile15, was hiermit durch Bezugnahme Bestandteil der vorliegenden Anmeldung sei.

Die Reaktion kann beispielsweise abgebrochen werden, wie dort von Seite 31, Zeile 19 bis Seite 31, Zeile 31 beschrieben und die Aufarbeitung erfolgen wie dort beschrieben von Seite 31, Zeile 33 bis Seite 32, Zeile 40, was hiermit jeweils durch Bezugnahme Bestandteil der vorliegenden Anmeldung sei.

Die Reaktion kann alternativ und bevorzugt erfolgen wie in der WO 2005 / 087828 für Ammonium-alpha-hydroxycarboxylat-Katalysatoren beschrieben. Die Reaktion kann beispielsweise abgebrochen werden, wie dort von Seite 11, Zeile 12 bis Seite 12, Zeile 5 beschrieben, was hiermit durch Bezugnahme Bestandteil der vorliegenden Anmeldung sei.

Die Reaktion kann alternativ erfolgen wie in der CN 10178994A oder CN 101805304 beschrieben.

Bei thermisch labilen Katalysatoren ist es weiterhin auch möglich, die Reaktion abzubrechen durch Erhitzen des Reaktionsgemischs auf eine Temperatur oberhalb von mindestens 80°C, bevorzugt mindestens 100 °C, besonders bevorzugt mindestens 120 °C. Dafür reicht in der Regel bereits die Erwärmung des Reaktionsgemischs, wie sie zur Abtrennung des unumgesetzten Isocyanats durch Destillation in der Aufarbeitung erforderlich ist.

Sowohl bei thermisch nicht-labilen als auch bei thermisch labilen Katalysatoren besteht die Möglichkeit, durch Zugabe von Deaktivatoren die Reaktion bei niedrigeren Temperaturen abzubrechen. Geeignete Deaktivatoren sind beispielsweise Chlorwasserstoff, Phosphorsäure, organische Phosphate, wie Dibutylphosphat oder Diethylhexylphosphat, und Carbamate wie Hydroxyalkylcarbamat.

Diese Verbindungen werden pur oder verdünnt in geeigneter Konzentration, die zum Reaktionsabbruch erforderlich ist, zugegeben.

Erfindungsgemäß antiflockulierend wirkende Additive sind:
Silylester (B) ausgewählt aus der Gruppe aus Phosphorsäuresilylester und Phosphonsäuresilylester.

Bevorzugt sind als Silylester (B) folgende Verbindungen:

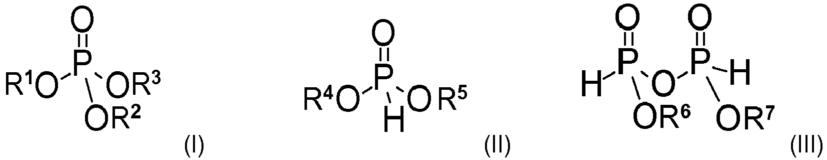

Darin bedeuten die Reste R¹ bis R⁷ jeweils unabhängig voneinander Trialkylsilylgruppe, Alkylgruppe oder Wasserstoff, wobei jede Verbindung mindestens eine Trialkylsilylgruppe enthalten muss. Bevorzugt sind die Reste R¹ bis R⁷ jeweils Trialkylsilylgruppen oder Alkylgruppen, wobei jede Verbindung mindestens eine Trialkylsilylgruppe enthalten muss. Besonders bevorzugt sind alle Reste R¹ bis R⁷ Trialkylsilylgruppen.

Die Alkylgruppen und die Alkylgruppen der Trialkylsilylgruppen sind bevorzugt C₁- bis C₁₈-Alkylgruppen.
Bevorzugt sind die Alkylgruppen der Trialkylsilylgruppen gleich.
Bevorzugt sind die Alkylgruppen der Trialkylsilylgruppen Methyl bzw. Ethyl, besonders bevorzugt Methyl.
Tris-(trimethylsilyl)-phosphorsäure ist die besonders bevorzugte Spezies.

Besonders bevorzugte Silylester (B) sind Phosphorsäuresilylester, insbesondere bevorzugt Phosphorsäure-tris-(silyl)-ester, ganz besonders bevorzugt Phosphorsäure-tris-(trimethylsilyl)-ester.

Die Silylester (B) werden in Mengen bezogen auf das Polyisocyanat (A) von 1 bis 250 Gew.ppm, bevorzugt von 5 bis 250 Gew.ppm, besonders bevorzugt von 10 bis 200 Gew.ppm zugesetzt, ganz besonders bevorzugt 10 bis 150 Gew.ppm.

Bevorzugt wird der Silylester (B) unterstöchiometrisch in Bezug auf die in dem Polyisocyanatgemisch vorliegende Wassermenge eingesetzt.

Als Lösungsmittel (C) für die Polyisocyanatkomponente, wie auch die Bindemittel- und ggf. weitere Komponenten einsetzbar sind solche, die keine gegenüber Isocyanatgruppen oder verkappten Isocyanatgruppen reaktiven Gruppen aufweisen und in denen die Polyisocyanate zu mindestens 10 Gew%, bevorzugt zu mindestens 25, besonders bevorzugt zu mindestens 50, ganz besonders bevorzugt zu mindestens 75, insbesondere zu mindestens 90 und speziell zu mindestens 95 Gew% löslich sind.

Beispiele für derartige Lösungsmittel sind aromatische (einschließlich alkylierter Benzole und Naphthaline) und/oder (cyclo)aliphatische Kohlenwasserstoffe und deren Gemische, Ketone, Ester, alkoxylierte Alkansäurealkylester, Ether, Etherester, respektive Gemische der Lösungsmittel.

Als aromatische Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), 150 (Siedebereich etwa 182 - 207 °C) und 200 (CAS-Nr. 64742-94-5), sowie die Shellsol®-Marken der Firma Shell, Caromax® (z.B. Caromax® 18) der Firma Petrochem Carless und Hydrosol der Firma DHC (z.B. als Hydrosol® A 170). Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen.

Der Gehalt an aliphatischen Kohlenwasserstoffen beträgt in der Regel weniger als 5, bevorzugt weniger als 2,5 und besonders bevorzugt weniger als 1 Gew%.

Ester sind beispielsweise n-Butylacetat, Ethylacetat, 1-Methoxypropylacetat-2 und 2-Methoxy-ethylacetat.

Ether sind beispielsweise THF, Dioxan sowie die Dimethyl-, -ethyl- oder -n-butylether von Ethylenglykol, Diethylenglykol, Triethylenglykol, Propylenglykol, Dipropylenglykol oder Tripropylenglykol.

Ketone sind beispielsweise Aceton, Diethylketon, Ethylmethylketon, Isobutylmethylketon, Methylamylketon und tert.-Butylmethylketon.

Etherester sind beispielsweise Ethylethoxypropionat EEP, Methoxymethylacetat, Butoxyethylacetat BGA, Ethoxy-1-methylethylacetat, Methoxy-1-methyl-ethylacetat.

Bevorzugte Lösungsmittel (C) sind Xylol, n-Butylacetat, Ethylacetat, 1-Methoxypropylacetat-2, 2-Methoxy-ethylacetat, sowie deren Gemische, insbesondere mit den oben aufgeführten aromatischen Kohlenwasserstoffgemischen, insbesondere Xylol und Solvesso® 100.

Derartige Gemische können im Volumenverhältnis 5:1 bis 1:5 erstellt werden, bevorzugt im Volumenverhältnis 4:1 bis 1:4, besonders bevorzugt im Volumenverhältnis 3:1 bis 1:3 und ganz besonders bevorzugt im Volumenverhältnis 2:1 bis 1:2.
Bevorzugte Beispiele sind Butylacetat/Xylol, Methoxypropylacetat/Xylol 1:1, Butylacetat/Solventnaphtha 100 1:1, Butylacetat/Solvesso® 100 1:2 und Kristallöl 30/Shellsol® A 3:1.
Besonders bevorzugt sind Butylacetat, 1-Methoxypropylacetat-2, Methylamylketon, Xylol und Solvesso® 100.
Bevorzugt werden die antiflockulierend wirkenden Additive homogen verteilt, bevorzugt unter Nutzung von Mischvorrichtungen.

Die antiflockulierend wirkenden Additive können auf vielfältige Art zugegeben werden, beispielsweise tel quel, in Lösungsmitteln (C) und/oder anderen Additiven, insbesondere auch in anderen Lösungsmittel, in dem es sich bevorzugt gut lösen sollte, wie beispielsweise Dialkyldicarbonsäureester wie Dioctyladipinsäureester und Dinonyladipinsäureester, Phosphorsäuretrialkylestern, Phthalsäureester und kernhydrierte Phthalsäureester, wie beispielsweise Cyclohexan-1,2- dicarbonsäurediisononylester, oder in Alkoholen. Aromatische Diester sind weniger bevorzugt.

Die antiflockulierend wirkenden Additive können beispielsweise eingebracht werden
- zu dem reinen Polyisocyanat
- zur einem verkaufsfähigen Polyisocyanat in Lösungsmittel,
- in einer Polyisocyanatkomponente, z.B. bestehend aus Polyisocyanat, Lösungsmittel und Additiven. Solch eine verkaufsfähige Polyisocyanatkomponente kann mit einer entsprechenden Polyolkomponente ohne weitere Zugaben, ggf. nach getrennter Lagerung, durch Abmischung der beiden Komponenten zur Lackierung eingesetzt werden.

Bevorzugt sind Additive, die in flüssiger Form vorliegen, oder mit geringen und kurzzeitig wirkenden Scherkräften eingerührt werden können.
Die antiflockulierend wirkenden Additive können zum Polyisocyanat bei erhöhter Temperatur direkt nach der Destillation bei ca. 170 °C bis Umgebungstemperatur, bei festen Additiven bevorzugt bei einer Temperatur über ihrem Schmelzpunkt, oder auch fest bei dann erhöhtem Aufwand zur Homogenisierung zugegeben werden.
In einer speziellen Form werden sie batchweise, ggf. in Lösungsmittel, in weniger als einer Stunde zwischen Raumtemperatur und 70 °C in einem Rührkessel mit dem Polyisocyanat vermischt.
In einer anderen speziellen Form werden sie, ggf. in Lösungsmittel, zwischen Raumtemperatur und 70 °C kontinuierlich in einem statischen Mischer zugegeben. Bevorzugt sind Temperaturen unter 35 °C, insbesondere bei Raumtemperatur.

Ein Vorlösen in Lösungsmittel kann insbesondere bei festen antiflockulierend wirkenden Additiven mit z.T. hohen Schmelzpunkten und schlechten Löslichkeiten in Polyisocyanat und Lösungsmittel sinnvoll sein.

Eine weitere Anwendungsform ist das Ansetzen einer Stammlösung, z.B. in Lösungsmittel, Additivgemischen und / oder Polyisocyanat.

Die Mengen in denen die Antiflockulanzien zugegeben werden, sind in den unten genannten Beispielen üblicherweise signifikant unterstöchiometrisch in Bezug auf die Gesamtwassermenge in der Mischung, respektive in Polyisocyanatkomponenten für die Lackapplikation, die ggf. noch zusätzliche Mengen an Lösungsmittel enthalten können.

In einer bevorzugten Form ist es vorteilhaft aus den oben genannten und weiteren Gründen, die minimal notwendige Menge Additiv zum Polyisocyanat zu geben mit der eine Flockulation, z.B. für ein halbes Jahr ausreichend unterbunden wird. Weitere Gründe für die Festlegung einer minimal notwendigen Menge sind, dass manche der Additive weitere funktionelle Wirkung in Polyurethansystemen haben können. Die Additive könnten in Wechselwirkung zu Lewis-Säure-Katalysatoren oder mit basischen Additiven wie UV-Stabilisatoren oder Aminkatalysatoren treten, und dabei schwer lösliche Salze bilden oder die Wirkung von Additiven aufheben. Von daher ist es vorteilhaft die Additivmenge möglichst gering zu halten, und ggf. abhängig von sonstigen Additiven selektiv auszuwählen.

Häufig gilt, dass die Flockulationsneigung mit zunehmender Verdünnung durch das Lösungsmittel zunimmt, insbesondere bei Lösungsmittelgehältern über 50 %, insbesondere bei über 60 %.

Des Weiteren kann das mit Additiv versetzte Polyisocyanatgemisch optional ein Urethanisierungskatalysator enthalten. Dabei kann es sich zum Beispiel um ein Amin oder eine metallorganische Verbindung halten.

Bevorzugt handelt es sich um eine Lewis-Säure.

Aminkatalysatoren sind beispielsweise trifunktionelle cycloaliphatische oder aromatische Amine mit Stickstoff im Heterozyklus. Beispiele dafür sind N-Methylmorpholin, N-Methyl piperidin, Pyrrolidin, Chinuclidin oder 1,4-Di-aza-bicyclo-[2.2.2]-octan, Diazabicycloundecan.

Metallorganische Verbindung sind beispielsweise, wie beispielsweise Zinn-(IV)- und Zinn-(II)-salze von organischen Carbonsäuren, z.B. Zinn-(II)-diacetat, Zinn-(II)-dioctoat, Zinn-(II)-bis(ethylhexanoat) und Zinn-(II)-dilaurat. Zudem können Zink-(II)-Salze eingesetzt werden, wie beispielsweise Zink-(II)-dioctoat, -di-neo-octanoat, -diacetat, oder - oxalat. Auch Metallkomplexe wie Acetylacetonate des Eisens, Titans, Aluminiums, Zirkons, Mangans, Nickels, Zinks und Cobalts sind möglich. Weitere Metallkatalysatoren werden von Blank et al. in Progress in Organic Coatings, 1999, Vol. 35, Seiten 19-29 beschrieben.

Dialkylzinn-(IV)-salze von organischen Carbonsäuren sind z.B. Dimethylzinn-diacetat, Dibutylzinn-diacetat, Dibutylzinn-dibutyrat, Dibutylzinn-bis(2-ethylhexanoat), Dibutylzinn-dilaurat, Dibutylzinn-maleat, Dioctylzinn-dilaurat und Dioctylzinn-diacetat. Bevorzugt sind Dibutylzinn-diacetat und Dibutylzinn-dilaurat. Zinnsalze sind aus toxikologischen Gründen weniger bevorzugt, werden aber in der Praxis immer noch häufig eingesetzt.

Weitere bevorzugte Lewis-saure organische Metallverbindungen sind Zink-(II)-dioctoat, Zirkon-acetylacetonat und Zirkon-2,2,6,6-tetramethyl-3,5-heptandionat.

Auch Bismut- und Kobalt-Katalysatoren, Cer-Salze wie Ceroctoate, und Cäsiumsalze können als Katalysatoren eingesetzt werden.
Bismut-Katalysatoren sind insbesondere Bismut-carboxylate, insbesondere Bismut-octoate, -ethylhexanoate, -neodecanoate, oder -pivalate; beispielsweise K-KAT 348 und XK-601 von King Industries, TIB KAT 716, 716LA, 716XLA, 718, 720, 789 von TIB Chemicals und solchen von Shepherd Lausanne, sowie Katalysator-Mischungen von z.B. Bismut- und Zink-Organylen.

Als Cäsiumsalze kommen dabei solche Verbindungen in Betracht, in denen folgende Anionen eingesetzt werden: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₂²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻*,* PO₄³⁻, P₂O₇⁴⁻, (OCₙH₂ₙ₊₁)⁻, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht. Bevorzugt sind dabei Cäsiumcarboxylate, bei denen das Anion den Formeln (CₙH₂ₙ₋₁O₂)⁻ sowie (Cₙ₊₁H₂ₙ₋₂O₄)²⁻ mit n gleich 1 bis 20, gehorcht. Besonders bevorzugte Cäsiumsalze weisen als Anionen Monocarboxylate der allgemeinen Formel (CₙH₂ₙ₋₁O₂)⁻ auf, wobei n für die Zahlen 1 bis 20 steht. Hierbei sind insbesondere zu erwähnen Format, Acetat, Propionat, Hexanoat und 2-Ethylhexanoat.

Als weitere lacktypische Komponenten und/oder Additive können beispielsweise verwendet werden: Stabilisatoren, UV-Stabilisatoren wie UV-Absorber und geeignete Radikalfängern (insbesondere HALS-Verbindungen, Hindered Amin Light Stabilizer), Aktivatoren (Beschleuniger), Trockenmittel, Füllmittel, Pigmente, Farbstoffe, antistatische Agenzien, Flammschutzmittel, Verdicker, thixotrope Agenzien, oberflächenaktive Agenzien, Viskositätsmodifikatoren, Plastifizierer oder Chelatbildner. Bevorzugt sind UV-Stabilisatoren.

Stabilisatoren sind mindestens eine stabilisierend wirkende Verbindung, wobei mit "stabilisierend" die Eigenschaft bezeichnet ist, die Entwicklung einer Farbzahl und/oder der Viskosität des Polyisocyanats im Verlauf der Lagerung über einen gewissen Zeitraum gegenüber solchen entsprechenden Mischungen zu verringern, die keine stabilisierend wirkenden Verbindungen enthalten.
Die Stabilisierung kann sich sowohl auf das Polyisocyanat alleine beziehen, wie auch auf Vormischungen der Polyisocyanate mit weiteren lacktypischen Komponenten und/oder Additiven, optional unter Zugabe weiterer Komponenten. Dies beinhaltet in einer besonderen Ausführung die Lagerung einer dieser Komponenten vor der eigentlichen Lackapplizierung.

Bevorzugt sind diese stabilisierend wirkenden Verbindungen ausgewählt aus der Gruppe bestehend aus primären Antioxidantien (Radikalfänger), sekundären Antioxidantien (Verbindungen, die die Radikalbildung verhindern, insbesondere indem sie Peroxide abfangen und/oder zersetzen) und sauren Stabilisatoren (Bronsted-Säuren).

Bei den primären Antioxidantien handelt es sich bevorzugt um sterisch gehinderte Phenole. Derartige sterisch gehinderte Phenole sind beispielsweise beschrieben in WO 2008/116894, bevorzugt die dort von Seite 14, Zeile 10 bis Seite 16, Zeile 10 beschriebenen Verbindungen, was hiermit per Bezugnahme Bestandteil der vorliegenden Offenbarung sei.

Dabei handelt es sich bevorzugt um solche Phenole oder überbrückte Bisphenole, die an dem aromatischen Ring genau eine phenolische Hydroxygruppe aufweisen und besonders bevorzugt um solche, die in den ortho-Positionen, ganz besonders bevorzugt in ortho- und para-Position zur phenolischen Hydroxy-gruppe einen beliebigen Substituenten, bevorzugt eine Alkylgruppe aufweisen, insbesondere um Alkyl-3-(3,5-di-tert.-butyl-4-hydroxyphenyl)-propionate, respektive um substituierte Alkyl-Derivate solcher Verbindungen.
In Verbindung mit erfindungsgemäßen Additiven sind sie Teil der Erfindung, bevorzugt 2,6-Bis-tert.-butyl-4-methyl-phenol (BHT), 3-[3,5-di-tert-butyl-4-hydroxyphenyl]-propionsäureester im Allgemeinen, Pentaerythrit-tetrakis(3-(3,5-di-tert-butyl-4-hydroxyphenyl)propionat (CAS-Nr. 6683-19-8; z.B. Irganox® 1010), 3,3',3",5,5',5"-Hexa-tert-butyl-a,a',a"-(mesitylen-2,4,6-triyl)tri-p-kresol (CAS-Nr. 1709-70-2; z.B. Irganox® 1330), 1,3,5-Tris(3,5-di-tert-butyl-4-hydroxybenzyl)-1,3,5-triazin-2,4,6(1 H,3H,5H)-trion (CAS-Nr. 27676-62-6; z.B. Irganox® 3114), Isoctyl-3-(3,5-di-tert-butyl-4-hydroxyphenyl)-propionat (CAS-Nr. 146598-26-7, z.B. Irganox® 1135) und Octadecyl-3-(3,5-di-tert-butyl-4-hydroxyphenyl)-propionat (CAS-Nr. 2082-79-3, z.B. Irganox® 1076).

Besonders bevorzugt sind 2,6-Di-tert.-butyl-4-methylphenol (BHT); Isooctyl-3-(3,5-di-tert.-butyl-4-hydroxyphenyl)-propionat (CAS-Nr. 146598-26-7, z.B. Irganox® 1135), Octadecyl-3-(3,5-di-tert.-butyl-4-hydroxyphenyl)-propionat (CAS-Nr. 2082-79-3, Irganox® 1076) und Pentaerythrit-tetrakis(3-(3,5-di-tert.-butyl-4-hydroxyphenyl)-propionat (CAS-Nr. 6683-19-8; z.B. Irganox® 1010).

Solche Phenole können auch Bestandteile eines polyphenolischen Systems mit mehreren Phenol-Gruppen sein Pentaerythrit-tetrakis(3-(3,5-di-tert.-butyl-4-hydroxyphenyl)propionat (z.B. Irganox® 1010); Ethylen-bis(oxyethylen)-bis-(3-(5-tert.-butyl-4-hydroxy-m-tolyl)-propionat) (z.B. Irganox 245); 3,3',3",5,5',5"-Hexa-tert.-butyl-α,α',α"-(mesitylen-2,4,6-triyl)tri-p-kresol (z.B. Irganox® 1330); 1,3,5-Tris(3,5-di-tert.-butyl-4-hydroxybenzyl)-1,3,5-triazin-2,4,6(1 H,3H,5H)-trion (z.B. Irganox® 3114), jeweils Produkte der Ciba Spezialitätenchemie, jetzt BASF SE.

Die sekundären Antioxidantien sind bevorzugt ausgewählt aus der Gruppe bestehend aus Phosphoniten, Phosphonaten und Thioethern, bevorzugt aus Phosphoniten oder Phosphonaten.

Bevorzugte Phosphonite sind beschrieben in WO 2008/116894, dort besonders von Seite 11, Zeile 8 bis Seite 14, Zeile 8, was hiermit per Bezugnahme Bestandteil der vorliegenden Offenbarung sei.

Bevorzugte Phosphonate sind beschrieben in WO 2008/116895, dort besonders von Seite 10, Zeile 38 bis Seite 12, Zeile 41, was hiermit per Bezugnahme Bestandteil der vorliegenden Offenbarung sei.

Bevorzugte Thioether sind beschrieben in WO 2008/116893, dort besonders von Seite 11, Zeile 1 bis Seite 15, Zeile 37, was hiermit per Bezugnahme Bestandteil der vorliegenden Offenbarung sei.

Bei den sauren Stabilisatoren handelt es sich um Bronsted-Säuren, wie sie beschrieben sind in WO 2008/116894, dort besonders von Seite 17, Zeile 34 bis Seite 18, Zeile 23, was hiermit per Bezugnahme Bestandteil der vorliegenden Offenbarung sei.

Geeignete UV-Absorber umfassen Oxanilide, Triazine und Benzotriazole (letztere erhältlich z.B. als Tinuvin® -Marken der BASF SE) und Benzophenone (z.B. Chimassorb® 81 der BASF SE). Bevorzugt sind z.B. 95 % Benzolpropansäure, 3-(2H-Benzotriazol-2-yl)-5-(1,1-dimethylethyl)-4-hydroxy-, C7-9-verzweigte und lineare Alkylester; 5 % 1-Methoxy-2-propylacetat (z.B. Tinuvin® 384) und α-[3-[3-(2H-Benzotriazol-2-yl)-5-(1,1,-dimethylethyl)-4-hydroxyphenyl]-1-oxopropyl]-ω-hydroxypoly(oxo-1,2-ethanediyl) (z.B. Tinuvin® 1130), jeweils Produkte z.B. der BASF SE. DL-Alpha-Tocopherol, Tocopherol, Zimtsäurederivate und Cyanoacrylate können ebenfalls zu diesem Zweck eingesetzt werden.

Diese können allein oder zusammen mit geeigneten Radikalfängern, beispielsweise sterisch gehinderten Aminen (oft auch als HALS -oder HAS-Verbindungen bezeichnet; Hindered Amine (Light) Stabilizer) wie 2,2,6,6-Tetramethylpiperidin, 2,6-Di-tert.-butylpiperidin oder deren Derivaten, z. B. Bis-(2,2,6,6-tetra-methyl-4-piperidyl)-sebacinat, eingesetzt werden. Diese sind z.B. erhältlich als Tinuvin®- und Chimassorb®-Marken der BASF SE. Bevorzugt im gemeinsamen Einsatz mit Lewis-Säuren sind jedoch solche gehinderten Amine, die N-alkyliert sind, beispielsweise Bis (1,2,2,6,6-pentamethyl-4-piperidinyl)-[[3,5-bis(1,1-dimethylethyl)-4-hydroxyphenyl]-methyl]butylmalonat (z.B. Tinuvin® 144 der BASF SE); eine Mischung aus Bis(1,2,2,6,6-Pen-tamethyl-4-Piperidinyl)sebacat und Methyl(1,2,2,6,6-Pentamethyl-4-Piperidinyl)sebacat (z.B. Tinuvin® 292 der BASF SE); oder die N-(O-alkyliert) sind, wie z.B. Dekandisäure-bis-(2,2,6,6-tetramethyl-1-(octyloxy)-4-piperidinyl)-ester, Reaktionsprodukte mit 1,1-Dimethylethylhydroperoxyd und Oktan (z.B. Tinuvin® 123 der BASF SE), und speziell dem HALS-Triazin
2,4-Bis[N-butyl-N-(1-cyclohexyloxy-2,2,6,6,-tetramethylpiperidin-4-yl)amino]-6-(2-hydroxyethyl-amin)-1,3,5-triazin (z.B. Tinuvin® 152 der BASF SE). Säureunempfindliche Additive wie das Tinuvin® 152 sind dabei vorteilhaft.

Trockenmittel sind beispielsweise para-Toluolsulfonylisocyanat (z.B. Additive TI der Fa. Borchers / OMG) und Ethylorthoformat (z.B. Additiv OF der Fa. Borchers / OMG).

UV-Stabilisatoren werden üblicherweise in Mengen von 0,1 bis 5,0 Gew.-%, bezogen auf die in der Zubereitung enthaltenen festen Komponenten, eingesetzt.

Als Verdicker kommen neben radikalisch (ko)polymerisierten (Ko)Polymeren, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Pigmente im eigentlichen Sinne sind gemäß CD Römpp Chemie Lexikon - Version 1.0, Stuttgart/New York: Georg Thieme Verlag 1995 unter Verweis auf DIN 55943 partikelförmige "im Anwendungsmedium praktisch unlösliche, anorganische oder organische, bunte oder unbunte Farbmittel".

Praktisch unlöslich bedeutet dabei eine Löslichkeit bei 25 °C unter 1 g / 1000 g Anwendungsmedium, bevorzugt unter 0,5, besonders bevorzugt unter 0,25, ganz besonders bevorzugt unter 0,1 und insbesondere unter 0,05 g / 1000 g Anwendungsmedium.

Beispiele für Pigmente im eigentlichen Sinne umfassen beliebige Systeme von Absorptions- und/oder Effektpigmenten, bevorzugt Absorptionspigmente. Anzahl und Auswahl der Pigmentkomponenten sind dabei keinerlei Beschränkungen unterworfen. Sie können den jeweiligen Erfordernissen, beispielsweise dem gewünschten Farbeindruck, beliebig angepasst werden, beispielsweise wie in Schritt a) beschrieben. Beispielsweise können alle Pigmentkomponenten eines standardisierten Mischlacksystems zugrunde liegen.

Unter Effektpigmenten sind alle Pigmente zu verstehen, die einen plättchenförmigen Aufbau zeigen und einer Oberflächenbeschichtung spezielle dekorative Farbeffekte verleihen. Bei den Effektpigmenten handelt es sich beispielsweise um alle in der Fahrzeug- und Industrielackierung üblicherweise einsetzbaren effektgebenden Pigmente. Beispiele für derartige Effektpigmente sind reine Metallpigmente; wie z.B. Aluminium-, Eisen- oder Kupferpigmente; Interferenzpigmente, wie z.B. titandioxidbeschichteter Glimmer, eisenoxidbeschichteter Glimmer, mischoxidbeschichteter Glimmer (z.B. mit Titandioxid und Fe₂O₃ oder Titandioxid und Cr₂O₃), metalloxidbeschichtetes Aluminium, oder Flüssigkristallpigmente.

Bei den farbgebenden Absorptionspigmenten handelt es sich beispielsweise um übliche in der Lackindustrie einsetzbare organische oder anorganische Absorptionspigmente. Beispiele für organische Absorptionspigmente sind Azopigmente, Phthalocyanin-, Chinacridon- und Pyrrolopyrrolpigmente. Beispiele für anorganische Absorptionspigmente sind Eisenoxidpigmente, Titandioxid und Ruß.

Farbstoffe sind ebenfalls Farbmittel und unterscheiden sich von den Pigmenten durch ihre Löslichkeit im Anwendungsmedium, d.h. sie weisen bei 25 °C eine Löslichkeit über 1 g / 1000 g im Anwendungsmedium auf.

Beispiele für Farbstoffe sind Azo-, Azin-, Anthrachinon-, Acridin-, Cyanin-, Oxazin-, Polymethin-, Thiazin-, Triarylmethan-Farbstoffe. Diese Farbstoffe können Anwendung finden als basische oder kationische Farbstoffe, Beizen-, Direkt-, Dispersions-, Entwicklungs-, Küpen-, Metallkomplex-, Reaktiv-, Säure-, Schwefel-, Kupplungs- oder Substantive Farbstoffe.

Als koloristisch inerte Füllstoffe sind alle Stoffe/Verbindungen zu verstehen, die einerseits koloristisch unwirksam sind; d.h. die eine geringe Eigenabsorption zeigen und deren Brechzahl ähnlich der Brechzahl des Beschichtungsmediums ist, und die andererseits in der Lage sind, die Orientierung (parallele Ausrichtung) der Effektpigmente in der Oberflächenbeschichtung, d.h. im applizierten Lackfilm, zu beeinflussen, ferner Eigenschaften der Beschichtung oder der Beschichtungsmassen, beispielsweise Härte oder Rheologie. Im Folgenden sind beispielhaft einsetzbare inerte Stoffe/Verbindungen genannt, ohne jedoch den Begriff koloristisch inerte topologiebeeinflussende Füllstoffe auf diese Beispiele zu beschränken. Geeignete inerte Füllstoffe entsprechend der Definition können beispielsweise transparente oder semitransparente Füllstoffe oder Pigmente sein, wie z.B. Kieselgele, Blancfixe, Kieselgur, Talkum, Calciumcarbonate, Kaolin, Bariumsulfat, Magnesiumsilikat, Aluminiumsilikat, kristallines Siliziumdioxid, amorphe Kieselsäure, Aluminiumoxid, Mikrokugeln oder Mikrohohlkugeln z.B. aus Glas, Keramik oder Polymeren mit Größen von beispielsweise 0,1-50 µm. Weiterhin können als inerte Füllstoffe beliebige feste inerte organische Partikel, wie z.B. Harnstoff-Formaldehyd-Kondensations-produkte, mikronisiertes Polyolefinwachs und mikronisiertes Amidwachs, eingesetzt werden. Die inerten Füllstoffe können jeweils auch in Mischung eingesetzt werden. Bevorzugt wird jedoch jeweils nur ein Füllstoff eingesetzt.

Bevorzugte Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Die Polyisocyanatkomponente enthaltend ein Additiv mit antiflockulierender Wirkung kann, ggf. nach Lagerung, mit mindestens einer Bindemittelkomponente und ggf., weiteren Komponenten z.B. zu Lacken oder Klebstoffen umgesetzt werden.

Bei den Bindemitteln kann es sich beispielsweise um Polyacrylatpolyole, Polyesterpolyole, Polyetherpolyole, Polyurethanpolyole; Polyharnstoffpolyole; Polyesterpolyacrylatpolyole; Polyesterpolyurethanpolyole; Polyurethanpolyacrylatpolyole, Polyurethanmodifizierte Alkydharze; Fettsäuremodifizierte Polyesterpolyurethanpolyole, Kopolymerisate mit Allylethern, Propfpolymerisate aus den genannten Stoffgruppen mit z.B. unterschiedlichen Glasübergangstemperaturen, sowie Mischungen der genannten Bindemittel handeln. Bevorzugt sind Polyacrylatpolyole, Polyesterpolyole und Polyurethanpolyole.

Bevorzugte OH-Zahlen, gemessen gemäß DIN 53240-2 (potentiometrisch), sind 40-350 mg KOH/g Festharz für Polyester, bevorzugt 80-180 mg KOH/g Festharz, und 15-250 mg KOH/g Festharz für Polyacrylatole, bevorzugt 80-160 mg KOH/g.

Zusätzlich können die Bindemittel eine Säurezahl gemäß DIN EN ISO 3682 (potentiometrisch) bis 200 mg KOH/g, bevorzugt bis 150 und besonders bevorzugt bis 100 mg KOH/g aufweisen.

Besonders bevorzugte Bindemittel sind Polyacrylatpolyole und Polyesterole.

Polyacrylatpolyole weisen bevorzugt ein Molekulargewicht Mₙ von mindestens 500, besonders bevorzugt mindestens 1200 g/mol auf. Das Molekulargewicht Mₙ kann prinzipiell nach oben unbegrenzt sein, bevorzugt bis zu 50.000, besonders bevorzugt bis zu 20.000 g/mol, ganz besonders bevorzugt bis zu 10.000 g/mol und insbesondere bis zu 5.000 g/mol betragen.

Die hydroxyfunktionellen Monomere (siehe unten) werden in solchen Mengen bei der Kopolymerisation mitverwendet, dass die obengenannten Hydroxylzahlen der Polymere resultieren, die im Allgemeinen einem Hydroxygruppengehalt der Polymere von 0,5 bis 8, vorzugsweise 1 bis 5 Gew.-% entsprechen.

Dabei handelt es sich um hydroxygruppenhaltige Kopolymere aus mindestens einem hydroxy-gruppenhaltigen (Meth)acrylat mit mindestens einem weiteren polymerisationsfähigen Komonomer ausgewählt aus der Gruppe bestehend aus (Meth)acrylsäurealkylestern, Vinylaromaten, α, β-ungesättigten Carbonsäuren und anderen Monomeren.

Als (Meth)acrylsäurealkylestern genannt seien z.B. C₁-C₂₀-Alkyl(meth)acrylate, Vinylaromaten sind solche mit bis zu 20 C-Atomen, α,β-ungesättigte Carbonsäuren umfassen auch deren Anhydride und andere Monomere sind beispielsweise Vinylester von bis zu 20 C-Atomen enthaltenden Carbonsäuren, ethylenisch ungesättigte Nitrile, Vinylether von 1 bis 10 C-Atome enthaltenden Alkoholen und, weniger bevorzugt, aliphatische Kohlenwasserstoffe mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α, β-Ungesättigte Carbonsäuren und deren Anhydride können beispielsweise sein: Acrylsäure, Methacrylsäure, Fumarsäure, Crotonsäure, Itaconsäure, Maleinsäure oder Maleinsäureanhydrid, bevorzugt Acrylsäure.

Als hydroxyfunktionelle Monomere seien Monoester von α,β-ungesättigten Carbonsäuren, wie Acrylsäure, Methacrylsäure (in dieser Schrift kurz als "(Meth)acrylsäure" bezeichnet), mit Di- oder Polyolen erwähnt, die vorzugsweise 2 bis 20 C-Atome und wenigstens zwei Hydroxygruppen aufweisen, wie Ethylenglykol, Diethylenglykol, Triethylenglykol, 1,2-Propylenglykol, 1,3-Pro-pylenglykol, 1,1-Dimethyl-1,2-Ethandiol, Dipropylenglykol, Triethylenglykol, Tetraethylenglykol, Pentaethylenglykol, Tripropylenglykol, 1,4-Butandiol, 1,5-Pentandiol, Neopentylglykol, Hydroxypivalinsäureneopentylglykolester, 2-Ethyl-1,3-Propandiol, 2-Methyl-1,3-Propandiol, 2-Butyl-2-ethyl-1,3-Propandiol, 1,6-Hexandiol, 2-Methyl-1,5-pentandiol, 2-Ethyl-1,4-butandiol, 2-Ethyl-1,3-Hexandiol, 2,4-Diethyl-oktan-1,3-diol, 2,2-Bis(4-hydroxycyclohexyl)propan, 1,1-, 1,2-, 1,3- und 1,4-Bis(hydroxymethyl)-cyclohexan, 1,2-, 1,3- oder 1,4-Cyclohexandiol, Glycerin, Trimethylolethan, Trimethylolpropan, Trimethylolbutan, Pentaerythrit, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit, Isomalt, Poly-THF mit einem Molekulargewicht zwischen 162 und 4500, bevorzugt 250 bis 2000, Poly-1,3-propandiol oder Polypropylenglykol mit einem Molekulargewicht zwischen 134 und 2000 oder Polyethylenglykol mit einem Molekulargewicht zwischen 238 und 2000 sein.

Bevorzugt sind 2-Hydroxyethylacrylat, 2-Hydroxyethylmethacrylat, 2- oder 3-Hydroxypropyl-acrylat, 1,4-Butandiolmonoacrylat oder 3-(Acryloyloxy)-2-hydroxypropylacrylat und besonders bevorzugt 2-Hydroxyethylacrylat und/oder 2-Hydroxyethylmethacrylat.

Als vinylaromatische Verbindungen kommen z.B. Vinyltoluol, α-Butylstyrol, α-Methylstyrol,
4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar, weiterhin, ethylenisch ungesättigte Säuren, insbesondere Carbonsäuren, Säureanhydride oder Säureamide, sowie Vinylimidazol. Auch Epoxidgruppen aufweisende Komonomere wie z.B. Glycidylacrylat oder -methacrylat oder Monomere wie N-Methoxymethylacrylamid oder -methacrylamid können in geringen Mengen mitverwendet werden.

Bevorzugt sind Ester der Acrylsäure bzw. der Methacrylsäure mit 1 bis 18, vorzugsweise 1 bis 8 Kohlenstoffatomen im Alkoholrest wie z.B. Methylacrylat. Ethylacrylat, Iso-propylacrylat, n-Pro-pylacrylat, n-Butylacrylat, 2-Ethylhexylacrylat, n-Stearylacrylat, die diesen Acrylaten entsprechenden Methacrylate, Styrol, alkylsubstituierte Styrole, Acrylnitril, Methacrylnitril, Vinylacetat oder Vinylstearat bzw. beliebige Gemische derartiger Monomere.

Die Hydroxygruppen tragenden Monomere werden in die Kopolymerisation der Hydroxygruppen tragenden (Meth)Acrylate im Gemisch mit anderen polymerisierbaren, bevorzugt radikalisch polymerisierbaren Monomeren, eingesetzt, bevorzugt solche, welche zu mehr als 50 Gew% aus C₁-C₂₀-, bevorzugt C₁- bis C₄-Alkyl(meth)acrylat, (Meth)acrylsäure, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymere, die neben den Hydroxygruppen tragenden Monomeren zu mehr als 60 Gew% aus C₁-C₁₀-Alkyl(meth)acrylaten, Styrol und dessen Derivaten oder deren Mischungen bestehen.

Die Herstellung der Polymerisate kann durch Polymerisation nach üblichen Verfahren durchgeführt werden. Vorzugsweise erfolgt die Herstellung der Polymerisate in einer Emulsionspolymerisation oder in organischer Lösung. Möglich sind kontinuierliche oder diskontinuierliche Polymerisationsverfahren. Von den diskontinuierlichen Verfahren sind das Batch- und das Zulaufverfahren zu nennen, wobei letzteres bevorzugt ist. Bei dem Zulaufverfahren wird das Lösungsmittel allein oder mit einem Teil des Monomergemischs vorgelegt, auf die Polymerisationstemperatur erwärmt, die Polymerisation im Fall einer Monomervorlage radikalisch gestartet und das restliche Monomergemisch zusammen mit einem Initiatorgemisch im Verlauf von 1 bis 10 Stunden, vorzugsweise 3 bis 6 Stunden, zudosiert. Optional wird anschließend noch nachaktiviert, um die Polymerisation bis zu einem Umsatz von mindestens 99 % durchzuführen.

Als Lösungsmittel kommen beispielsweise Aromaten, wie Solventnaphtha, Benzol, Toluol, Xylol, Chlorbenzol, Ester wie Ethylacetat, Butylacetat, Methylglykolacetat, Ethylglykolacetat, Methoxypropylacetat, Ether wie Butylglykol, Tetrahydrofuran, Dioxan, Ethylglykolether, Ketone wie Aceton, Methylethylketon, halogenhaltige Lösemittel wie Methylenchlorid oder Trichlormonofluorethan in Betracht.

Weitere Bindemittel sind z.B. Polyesterpolyole, wie sie durch Kondensation von Polycarbonsäuren, insbesondere Dicarbonsäuren mit Polyolen, insbesondere Diolen erhältlich sind. Um eine für die Polymerisation angemessene Funktionalität des Polyesterpolyols zu gewährleisten werden partiell auch Triole, Tetrole etc. wie auch Trisäuren, etc. eingesetzt.

Polyesterpolyole, sind z.B. aus Ullmanns Enzyklopädie der technischen Chemie, 4. Auflage, Band 19, S. 62 bis 65 bekannt. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, aromatisch oder heterocyclisch sein und optional, z.B. durch Halogenatome, substituiert und/oder ungesättigt sein. Als Beispiele hierfür seien genannt:
Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, Korksäure, Azelainsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäureanhydrid, dimere Fettsäuren, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, besonders bevorzugt Bernsteinsäure, Adipinsäure, Sebacinsäure und Dodecandicarbonsäure.

Als mehrwertige Alkohole kommen zur Herstellung der Polyesterole in Betracht 1,2-Propandiol, Ethylenglykol, 2,2-Dimethyl-1,2-Ethandiol, 1,3-Propandiol, 1,2-Butandiol, 1,3-Butandiol, 1,4-Bu-tandiol, 3-Methylpentan-1,5-diol, 2-Ethylhexan-1,3-diol, 2,4-Diethyloctan-1,3-diol, 1,6-Hexandiol, Poly-THF mit einer Molmasse zwischen 162 und 4500, bevorzugt 250 bis 2000, Poly-1,3-pro-pandiol mit einer Molmasse zwischen 134 und 1178, Poly-1,2-propandiol mit einer Molmasse zwischen 134 und 898, Polyethylenglykol mit einer Molmasse zwischen 106 und 458, Neopentylglykol, Hydroxypivalinsäureneopentylglykolester, 2-Ethyl-1,3-Propandiol, 2-Methyl-1,3-Pro-pandiol, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Trimethylolbutan, Trimethylolpropan, Trimethylolethan, Neopentylglykol, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit oder Isomalt, die optional wie oben beschrieben alkoxiliert sein können.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Bevorzugt sind Ethylenglykol, Butan-1,4-diol, Hexan-1,6-diol, Octan-1,8-diol und Dodecan-1,12-diol. Weiterhin bevorzugt ist Neopentylglykol.

Ferner kommen auch Polycarbonat-Diole in Betracht, wie sie z.B. durch Umsetzung von Phosgen mit einem Überschuss von den als Aufbaukomponenten für die Polyesterpolyole genannten niedermolekularen Alkohole erhalten werden können.

Geeignet sind auch Polyesterdiole auf Lacton-Basis, wobei es sich um Homo- oder Mischpolymerisate von Lactonen, bevorzugt um endständige Hydroxygruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, gamma-Butyrolacton und/oder Methyl-ε-caprolacton, 4-Hydroxybenzoesäure, 6-Hydroxy-2-naphthalinsäure oder Pivalolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

In Polyurethanlacken sind Molmassen Mₙ der Polyester von 800 - 4000 g/mol üblich, wobei die hier verwendeten Polyester nicht darauf beschränkt sind.

Weiterhin sind als Bindemittel auch Polyetherole geeignet, die durch Addition von Ethylenoxid, Propylenoxid und/oder Butylenoxid, bevorzugt Ethylenoxid und/oder Propylenoxid und besonders bevorzugt Ethylenoxid an H-aktive Komponenten hergestellt werden. Ebenso sind Polykondensate aus Butandiol geeignet. In Polyurethanlacken sind Molmassen der Polyether von 500-2000 g/mol üblich, wobei die hier verwendeten Polyether nicht darauf beschränkt sind.

Die Polymere können zumindest teilweise durch sogenannte Reaktivverdünner ersetzt werden. Dabei kann es sich um blockierte sekundäre oder primäre Amine (Aldimine und Ketime) oder um Verbindungen mit sterisch gehinderten und / oder elektronenarmen sekundären Aminogruppen handeln, beispielsweise Asparaginsäureester gemäß EP 403921 oder WO 2007/39133.

Nach Vermischen der Polyisocyanatkomponente mit einer Bindemittelkompontente und ggf. weiteren Komponenten wird das Lackgemisch bei Umgebungstemperatur bis 150 °C ausgehärtet.

Unter "Härtung" wird im Rahmen der vorliegenden Erfindung das Erzeugen einer klebfreien Beschichtung auf einem Substrat verstanden, indem man die auf das Substrat aufgetragene Beschichtungsmasse zumindest solange auf die oben angegebene Temperatur erwärmt, bis mindestens die gewünschte Klebfreiheit eingetreten ist.

In einer bevorzugten Form wird im Rahmen der vorliegenden Schrift unter einer Beschichtungsmasse eine Mischung zumindest zweier Komponenten (Bindemittel und Vernetzer) verstanden, die zum Beschichten mindestens eines Substrats zum Zwecke einer Ausbildung eines Films und, nach Härtung, einer klebfreien Beschichtung vorgesehen ist.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die optional enthaltenen flüchtigen Bestandteile der Beschichtungsmasse, optional unter Erhitzen, entfernt. Dieser Vorgang kann gewünschten falls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Koextrudieren erfolgen.

Die Dicke einer solchen zu härtenden Schicht kann von 0,1 µm bis mehrere mm betragen, bevorzugt von 1 bis 2.000 µm, besonders bevorzugt 5 bis 200 µm, ganz besonders bevorzugt von 5 bis 60 µm (bezogen auf den Lack im Zustand in dem das Lösungsmittel aus dem Lack entfernt ist).

Weiterhin sind auch Substrate, beschichtet mit einer erfindungsgemäßen Mehrschichtlackierung Gegenstand der vorliegenden Erfindung.

Besonders geeignet sind solche Polyurethanlacke für Anwendungen, in denen eine besonders hohe Applikationssicherheit, Außenwitterungsbeständigkeit, Optik, Lösemittel-, Chemikalien- und Wasserfestigkeit gefordert werden.

Die erhaltenen zweikomponentigen Beschichtungsmassen und Lackformulierungen eignen sich zum Beschichten von Substraten wie Holz, Holzfurnier, Papier, Pappe, Karton, Textil, Folie, Leder, Vlies, Kunststoffoberflächen, Glas, Keramik, mineralischen Baustoffen, wie Zement-Formsteine und Faserzementplatten oder Metallen, die jeweils optional vorbeschichtet bzw. vorbehandelt sein können.

Derartige Beschichtungsmassen eignen sich als oder in Innen- oder Außenbeschichtungen, also solche Anwendungen, die dem Tageslicht ausgesetzt sind, bevorzugt von Gebäudeteilen, Beschichtungen auf (Groß-)Fahrzeugen und Flugzeugen und industriellen Anwendungen, Nutzfahrzeuge im landwirtschaftlichen und Baubereich, Dekolackierungen, Brücken, Gebäuden, Strommasten, Tanks, Containern, Pipelines, Kraftwerken, chemischen Anlagen, Schiffen, Kränen, Pfählen, Spundwänden, Armaturen, Rohren, Fittings, Flanschen, Kupplungen, Hallen, Dächern und Baustahl, Möbeln, Fenstern, Türen, Parkett, Can-Coating und Coil-Coating, für Bodenbeläge, wie bei Parkdecks oder in Krankenhäusern, in Automobillacken als OEM und Refinish-Anwendung.

In einer bevorzugten Form werden derartige Beschichtungsmassen bei Temperaturen zwischen Umgebungstemperatur bis 80 °C, bevorzugt bis 60 °C, besonders bevorzugt bis 40 °C eingesetzt. Bevorzugt handelt es sich dabei um solche Gegenstände, die nicht bei hohen Temperaturen gehärtet werden können, wie große Maschinen, Flugzeuge, Großraumfahrzeuge und Refinish-Anwendungen. Als Umgebungstemperatur wird üblicherweise die Temperatur verstanden, in dem das beschichtete Substrat bestimmungsgemäß verwendet wird.

In einer anderen bevorzugten Anwendung wird das Lackgemisch bei 110-150 °C, bevorzugt bei 120-140 °C ausgehärtet (z.B. für OEM-Anwendungen).

Insbesondere werden die erfindungsgemäßen Beschichtungsmassen als Klar-, Basis- und Decklacke(n), Primern und Füllern eingesetzt.

Lange Lagerungen der Polyisocyanatkomponente sind insbesondere bei Refinish-, z.T. auch bei Industrieanwendungen üblich. Das mit antiflockulierend wirkenden Additiven versetzte Polyisocyanat respektive die Polyisocyanatkomponente kann natürlich auch für jede andere Anwendung eingesetzt werden.

### Beispiele:

### Einsatzstoffe:

### Polyisocyanate (A): Isocyanurat auf Basis Hexamethylendiisocyanat

Polyisocyanat (A1), Polyisocyanurat auf Basis Hexamethylendiisocyanat: Hexamethylendiisocyanat HDI wurde in Anwesenheit von 80 ppm Benzyltrimethylammonium-hydroxyisobutyrat als Katalysator bezogen auf Hexamethylendiisocyanat, 60%-ig in Ethylenglykol, in einer mehrstufigen Reaktorkaskade bei 115, 120 und 130 °C umgesetzt. Hexamethylendiisocyanat wurde in einem mehrstufigen Verfahren mit HDI-Rückführung abdestilliert. NCO-Gehalt des Produkts: 22,2 %, Viskosität: 2675 mPa*s.

### Polyisocyanat (A2): Polyisocyanurat auf Basis Hexamethylendiisocyanat

| | |
|---|---|
| Basonat HI 100 | BASF SE |

**Silylderivate:**

| | | |
|---|---|---|
| Tristrimethylsilylphosphat | Fa. Sigma-Aldrich | - Erfindungsgemäß |
| N,O-Bis-(trimethylsilyl)-acetamid | Fa. Sigma-Aldrich | - Nicht erfindungsgemäß |
| 1,3-Bis-(trimethylsilyl)-harnstoff | Fa. Sigma-Aldrich | - Nicht erfindungsgemäß |
| Hexamethyldisilazan | Fa. Sigma-Aldrich | - Nicht erfindungsgemäß |

### Lösungsmittel (C):

### Xylol

### Butylacetat

**Sterisch gehinderte Phenole:**

| | |
|---|---|
| Irganox® 1010: | Pentaerythrit-tetrakis(3-(3,5-di-tert-butyl-4-hydroxyphenyl)propionat, Fa. BASF SE |
| Irganox® 1076: | Octadecyl-3-(3,5-di-tert-butyl-4-hy-droxyphenyl)-propionat, Fa. BASF SE |
| Irganox® 1135: | Isooctyl-3-(3,5-di-tert-butyl-4-hydroxyphenyl)-propionat, Fa. BASF SE |

**Sekundäre Antioxidanzien:**

| | |
|---|---|
| Irgafos OPH | Fa. BASF SE |
| Triphenylphosphit | Fa. Alfa Aesar |
| Tributylphosphit | Fa. Sigma-Aldrich |

### Lagertest:

Zur Bestimmung der Flockulationsstabilität wurden Mischungen bestehend aus 30 % Polyisocyanat und 70 % Xylol / Butylacetat = 2:1, enthaltend 400 ppm Wasser auf Lösungsmittelgemisch, hergestellt. Additive wurden über die Lösungsmittel eingebracht. 50 g der Mischung wurden in 50 mL Schraubdeckelgefäßen ohne seitliche Beschriftung mit Stickstoff überströmend inertisiert und danach fest verschlossen bei 23 °C ± 5 °C / (50 % ± 10 % Luftfeuchtigkeit) in einem Klimaraum gelagert. Die Flockulation wurde in den ersten zwei Wochen wochentags täglich, nach drei Wochen, ab vier Wochen zweiwöchentlich visuell begutachtet.

Zur Beurteilung der Flockulationsstabilität wird der jeweils erste Tag angegeben bei dem eine der zwei folgenden Flockulationsstufen erreicht wird:
1 Leichte Flockulation: schwach erkennbar. Zum besseren Erkennen einer Trübung werden scheinbar klare Lösungen kurz leicht horizontal im Kreis gedreht. Manchmal ist dann eine leichte Flockulation besser als Schweif erkennbar.
2 Starke Flockulation, gut erkennbar, Bodensatz, Abbruch der Untersuchung

Die Bestimmung der Flockulation erfolgt zwar durch erfahrene Personen, aber eine gewisse Standardabweichung als Folge leicht unterschiedlicher Bedingungen (z.B. exakte Wassermenge im Lösungsmittel etc.) und bei der optischen Bestimmung einer leichten und schweren Trübung ist vorhanden. In verschiedenen Serien kann es zu leicht unterschiedlichen Ergebnissen von Mustern "gleicher" Zusammensetzung kommen, die aber in den Tageszahlen trotz allem vergleichbar, und im Verhältnis guter Beispiele zu schlechten Referenzen deutlich geringer sind.

Die Tageswerte für die zwei Flockulationsstufen werden für den besseren Vergleich zusätzlich noch zusammengezählt. Je höher die Tageswerte oder deren Summe desto besser die Flockulationsstabilität. Bei 210 Messtagen erfolgt automatisch ein Abbruch der Messung.

### Serie 1: Polyisocyanat (A1): Flockulationszeiten in Tagen nach maximal 210 Messtagen

| Fl./d | Additive (ppm) | 1 | 2 | Summe |
|---|---|---|---|---|
| V1 | 200 Irganox 1135 | 28 | 28 | 56 |
| B1 | 200 Irganox 1135 + 100 Tristrimethylsilylphosphat | 84 | 196 | 280 |
| V2 | 200 Triphenylphosphit | 2 | 98 | 100 |
| B2 | 200 Triphenylphosphit + 100 Tristrimethylsilylphosphat | 84 | 210 | 294 |
| V3 | 200 Irganox 1076 + 200 Triphenylphosphit | 12 | 21 | 33 |
| B3 | 200 Irganox 1076 + 200 Triphenylphosphit + 100 Tristrimethylsilylphosphat | 12 | 210 | 222 |
| V4 | 200 Irganox 1135 + 200 Triphenylphosphit | 6 | 28 | 34 |
| B4 | 200 Irganox 1135 + 200 Triphenylphosphit + 50 Tristrimethylsilylphosphat | 126 | 210 | 336 |
| B5 | 200 Irganox 1135 + 200 Triphenylphosphit + 100 Tristrimethylsilylphosphat | 84 | 210 | 294 |
| V5 | 200 Irganox 1076 + 200 Irgafos OPH | 28 | 28 | 56 |
| B6 | 200 Irganox 1076 + 200 Irgafos OPH + 100 Tristrimethylsilylphosphat | 56 | 182 | 238 |
| V6 | 200 Irganox 1010 + 200 Irgafos OPH | 42 | 42 | 84 |
| B7 | 200 Irganox 1010 + 200 ppm Irgafos OPH + 100 Tristrimethylsilylphosphat | 84 | 210 | 294 |

Ergebnis: Tristrimethylsilylphosphat verbessert die Flockulation in Kombination mit mindestens je einem Antioxidanz.

### Serie 2: Polyisocyanat (A2): Flockulationszeiten in Tagen nach maximal 210 Messtagen

| Fl./d | Additive (ppm) | 1 | 2 | Summe |
|---|---|---|---|---|
| V7 | ohne | 11 | 21 | 32 |
| B8 | 50 Tristrimethylsilylphosphat | 56 | 210 | 266 |
| B9 | 100 Tristrimethylsilylphosphat | 28 | 210 | 238 |
| V8 | 200 Irganox 1135 | 8 | 21 | 29 |
| B10 | 200 Irganox 1135 + 100 Tristrimethylsilylphosphat | 28 | 210 | 238 |
| V9 | 200 Triphenylphosphit | 14 | 21 | 35 |
| B11 | 200 Triphenylphosphit + 100 Tristrimethylsilylphosphat | 56 | 154 | 210 |
| V10 | 200 Irgafos OPH | 21 | 21 | 42 |
| B12 | 200 Irgafos OPH + 100 Tristrimethylsilylphosphat | 56 | 210 | 266 |
| V11 | 200 Irganox 1135 + 200 Tributylphosphit | 21 | 21 | 42 |
| B13 | 200 Irganox. 1135 + 200 Tributylphosphit + 100 Tristrimethylsilylphosphat | 13 | 210 | 223 |
| V12 | 200 Irganox 1010 + 200 Triphenylphosphit | 28 | 42 | 70 |
| B14 | 200 Irganox 1010 + 200 Triphenylphosphit + 100 Tristrimethylsilylphosphat | 28 | 196 | 224 |
| V13 | 200 Irganox 1076 + 200 Triphenylphosphit | 12 | 42 | 54 |
| B15 | 200 Irganox 1076 + 200 Triphenylphosphit + 100 Tristrimethylsilylphosphat | 9 | 126 | 135 |
| V14 | 200 Irganox 1135 + 200 Triphenylphosphit | 8 | 21 | 29 |
| B16 | 200 Irganox 1135 + 200 Triphenylphosphit + 50 Tristrimethylsilylphosphat | 8 | 210 | 218 |
| V15 | 200 Irganox 1135 + 200 Triphenylphosphit + 300 Tristrimethylsilylphosphat | 7 | 8 | 15 |
| V16 | 200 Irganox 1135 + 200 Triphenylphosphit + 1000 Tristrimethylsilylphosphat | 5 | 5 | 10 |

Ergebnis: (50 und) 100 ppm Tristrimethylsilylphosphat verbessern die Flockulation ohne Antioxidanz, mit je einem Antioxidanz und mit je zweien.

300 und 1000 ppm Tristrimethylsilylphosphat wirken sich in Kombination mit 200 Irganox 1135 + 200 Triphenylphosphit negativ aus.

### Serie 3: Polyisocyanat (A1): Flockulationszeiten in Tagen nach maximal 126 Messtagen (Abbruch wegen der Flockulationen)

| Fl./d | Additive (ppm) | 1 | 2 | Summe |
|---|---|---|---|---|
| V17 | 200 Irganox 1135 + 200 Irgafos OPH | 21 | 21 | 42 |
| V18 | 200 Irganox 1135 + 200 Irgafos OPH + 100 N,O-Bis-(trimethylsilyl)-acetamid | 5 | 13 | 18 |
| V19 | 200 Irganox 1135 + 200 Irgafos OPH + 100 1,3-Bis-(trimethylsilyl)-harnstoff | 9 | 13 | 22 |
| V20 | 200 Irganox 1135 + 200 Irgafos OPH + 100 Hexamethyldisilazan | 9 | 13 | 22 |
| B17 | 200 Irganox 1135 + 200 Irgafos OPH + 100 Tristrimethylsilylphosphat | 12 | 126 | 138 |

Ergebnis: 100 ppm Tristrimethylsilylphosphat verbessern die Flockulation; N,O-Bis-(trimethylsilyl)-acetamid, Hexamethyldisilazan und 1,3-Bis-(trimethylsilyl)-harnstoff nicht.

### Serie 4: Polyisocyanat (A1): Flockulationszeiten in Tagen nach maximal 210 Messtagen

| Fl./d | Additive (ppm) | 1 | 2 | Summe |
|---|---|---|---|---|
| V21 | 200 Irganox 1135 + 200 Irgafos OPH | 7 | 14 | 21 |
| V22 | 200 Irganox 1135 + 200 Irgafos OPH + 1 Tristrimethylsilylphosphat | 7 | 11 | 18 |
| B18 | 200 Irganox 1135 + 200 Irgafos OPH + 5 Tristrimethylsilylphosphat | 14 | 56 | 70 |
| B19 | 200 Irganox 1135 + 200 Irgafos OPH + 10 Tristrimethylsilylphosphat | 126 | 210 | 336 |
| B20 | 200 Irganox 1135 + 200 Irgafos OPH + 20 Tristrimethylsilylphosphat | 14 | 210 | 224 |
| B21 | 200 Irganox 1135 + 200 Irgafos OPH + 50 Tristrimethylsilylphosphat | 14 | 182 | 196 |
| B22 | 200 Irganox 1135 + 200 Irgafos OPH + 100 Tristrimethylsilylphosphat | 2 | 182 | 184 |
| V23 | 200 Irganox 1135 + 200 Irgafos OPH + 300 Tristrimethylsilylphosphat | 2 | 9 | 11 |
| V24 | 200 Irganox 1135 + 200 Irgafos OPH + 1000 Tristrimethylsilylphosphat | 2 | 7 | 9 |

Ergebnis: 5 ppm Tristrimethylsilylphosphat verbessern die Flockulation leicht, 10; 20; 50 und 100 ppm Tristrimethylsilylphosphat sehr deutlich. 300 und 1000 ppm wirken sich nicht positiv bzw. negativ aus.

### Serie 5: Polyisocyanat (A2): Flockulationszeiten in Tagen nach maximal 210 Messtagen

| Fl./d | Additive (ppm) | 1 | 2 | Summe |
|---|---|---|---|---|
| V25 | 200 Irganox 1135 + 200 Irgafos OPH | 7 | 21 | 28 |
| B23 | 200 Irganox 1135 + 200 Irgafos OPH + 1 Tristrimethylsilylphosphat | 7 | 56 | 63 |
| B24 | 200 Irganox 1135 + 200 Irgafos OPH + 5 Tristrimethylsilylphosphat | 56 | 210 | 266 |
| B25 | 200 Irganox 1135 + 200 Irgafos OPH + 10 Tristrimethylsilylphosphat | 21 | 210 | 231 |
| B26 | 200 Irganox 1135 + 200 Irgafos OPH + 20 Tristrimethylsilylphosphat | 21 | 210 | 231 |
| B27 | 200 Irganox 1135 + 200 Irgafos OPH + 50 Tristrimethylsilylphosphat | 7 | 210 | 217 |
| B28 | 200 Irganox 1135 + 200 Irgafos OPH + 100 Tristrimethylsilylphosphat | 7 | 210 | 217 |
| V26 | 200 Irganox 1135 + 200 Irgafos OPH + 300 Tristrimethylsilylphosphat | 7 | 8 | 15 |
| V27 | 200 Irganox 1135 + 200 Irgafos OPH + 1000 Tristrimethylsilylphosphat | 7 | 7 | 14 |

Ergebnis: 1 ppm Tristrimethylsilylphosphat verbessert die Flockulation leicht. Ab 5 ppm Tristrimethylsilylphosphat ist die Verbesserung sehr deutlich. 300 und 1000 ppm Tristrimethylsilylphosphat wirken sich wieder negativ aus.

## Patentansprüche

1. Verwendung von Additiven zur Verringerung von Flockulation und/oder Niederschlagsbildung von Polyisocyanatgemischen, welche mindestens ein Lösungsmittel (C) und mindestens ein Polyisocyanat (A) enthalten,
wobei die Additive ausgewählt sind aus der Gruppe von Silylestern (B) bestehend aus Phosphorsäuresilylester und Phosphonsäuresilylester und in einer Menge von 1 bis 250 Gew.ppm bezogen auf das mindestens eine Polyisocyanat (A) eingesetzt werden.

2. Verwendung von Additiven nach Anspruch 1 **dadurch gekennzeichnet, dass** der Silylester (B) ein Phosphorsäuresilylester ist.

3. Verwendung von Additiven nach Anspruch 1 **dadurch gekennzeichnet, dass** der Silylester (B) ein Phosphorsäure-tris-(silyl)-ester, bevorzugt ein Phosphorsäuretris-(trimethylsilyl)-ester ist.

4. Verwendung von Additiven nach einem der vorhergehenden Ansprüche **dadurch gekennzeichnet, dass** der Silylester (B) in einer Menge von 5-250 Gew.ppm, bevorzugt von 10-200 Gew.ppm, bezogen auf das mindestens eine Polyisocyanat (A) eingesetzt wird.

5. Verwendung von Additiven nach einem der vorhergehenden Ansprüche **dadurch gekennzeichnet, dass** der Silylester (B) unterstöchiometrisch in Bezug auf die in dem Polyisocyanatgemisch vorliegende Wassermenge eingesetzt wird.

6. Verwendung von Additiven nach einem der vorhergehenden Ansprüche **dadurch gekennzeichnet, dass** das mindestens eine Polyisocyanat (A) einen NCO-Gehalt von mindestens 15 Gew.%, bevorzugt mindestens 20 Gew.% aufweist.

7. Verwendung von Additiven nach einem der vorhergehenden Ansprüche **dadurch gekennzeichnet, dass** das mindestens eine Polyisocyanat (A) (cyclo)aliphatisch ist.

8. Verwendung von Additiven nach einem der vorhergehenden Ansprüche **dadurch gekennzeichnet, dass** das mindestens eine Polyisocyanat (A) auf 1,6-Hexamethylendiisocyanat, 1,5-Pentamethylendiisocyanat, Isophorondiisocyanat, 1,3-Bis(isocyanatomethyl)cyclo-hexan, 4,4'- Di(isocyanatocyclohexyl)methan und/oder 2,4'-Di(isocyanatocyclohexyl)methan als Monomeren basiert.

9. Verwendung von Additiven nach einem der vorhergehenden Ansprüche **dadurch gekennzeichnet, dass** das mindestens eine Polyisocyanat (A) Isocyanurat-, Iminooxadiazindion-, Biuret- oder Allophanat-(gegebenenfalls mit Urethan-)-Strukturen aufweist.

10. Verwendung von Additiven nach einem der vorhergehenden Ansprüche **dadurch gekennzeichnet, dass** die mit Additiv versetzten Polyisocyanatgemische zusätzlich mindestens ein Phenol oder überbrücktes Bisphenol enthalten, das an dem aromatischen Ring je genau eine phenolische Hydroxygruppe aufweist und in den ortho-Positionen zur phenolischen Hydroxygruppe Alkylgruppen aufweist, bevorzugt jeweils eine tert.-Butylgruppen aufweist.

11. Verwendung von Additiven nach einem der vorhergehenden Ansprüche, **dadurch gekennzeichnet, dass** die mit Additiv versetzten Polyisocyanatgemische zusätzlich mindestens eine Lewis-Säure enthalten.

12. Verfahren zur Stabilisierung von Polyisocyanatgemischen, welche mindestens ein Lösungsmittel (C) und mindestens ein Polyisocyanat (A) enthalten, gegen Flockulation während der Lagerung, **dadurch gekennzeichnet, dass** man mindestens ein Additiv ausgewählt aus der Gruppe von Silylestern (B) bestehend aus Phosphorsäuresilylester und Phosphonsäuresilylester in einer Menge von 1 bis 250 Gew.ppm bezogen auf das mindestens eine Polyisocyanat (A) zum Polyisocyanatgemisch gibt.

13. Verfahren zur Stabilisierung von Polyisocyanatgemischen gegen Flockulation nach Anspruch 12, **dadurch gekennzeichnet, dass** die Polyisocyanatgemische Lewis-Säuren wie beispielsweise Zinn-, Zink, Bismut-, Titan, Zirkonium-organische Verbindungen als Katalysatoren und/oder Antioxidantien und/oder andere übliche Lackadditive enthalten.

14. Verfahren zur Beschichtung von Substraten mit, gegen Flockulation während der Lagerung in Lösungsmitteln stabilisierten Polyisocyanatgemischen, welche mindestens ein Polyisocyanat (A) enthalten, **dadurch gekennzeichnet, dass** man mindestens ein Additiv ausgewählt aus der Gruppe von Silylestern (B) bestehend aus Phosphorsäuresilylester und Phosphonsäuresilylester, zum Polyisocyanatgemisch in einer Menge von 1 bis 250 Gew.ppm bezogen auf das mindestens eine Polyisocyanat (A) zusetzt und dieses mit mindestens einem Lösungsmittel (C) und optional anderen Additiven versetzt, lagert, und in einem Folgeschritt mit mindestens einer Bindemittel enthaltenden Komponente versetzt und anschließend auf ein Substrat aufbringt.

15. Verfahren gemäß Anspruch 14, **dadurch gekennzeichnet, dass** die Bindemittel enthaltende Komponente Verbindungen ausgewählt aus der Gruppe bestehend aus Polyacrylatpolyolen, Polyesterpolyolen, Polyurethanpolyolen, Polycarbonatpolyolen und Polyetherpolyolen enthalten.

16. Verwendung von Polyisocyanatkomponenten erhalten nach einem der Ansprüche 14 oder 15 in Beschichtungsmitteln in Grundierungen, Füllern, pigmentierten Decklacken, Basislacken und Klarlacken im Bereich Automobil-, Autoreparatur-, Großfahrzeug-, Kunststoff- und Holzlackierung, bei Nutzfahrzeugen im landwirtschaftlichen und Baubereich sowie als Härter in Klebstoffen und Dichtungsmassen.
